Europäisches Patentamt

European Patent Office

Office européen des brevets

(19)

(11) Numéro de publication: **0 090 150**
**B1**

(12) **FASCICULE DE BREVET EUROPEEN**

(45) Date de publication du fascicule du brevet: **20.11.86**

(51) Int. Cl.⁴: **C 07 D 307/60, B 01 J 23/44**

(21) Numéro de dépôt: **83101107.7**

(22) Date de dépôt: **05.02.83**

(54) Procédé pour la préparation de 4-hydroxy-2,5-diméthyl-2,3-dihydrofuran-3-one.

(30) Priorité: **26.03.82 CH 1869/82**

(43) Date de publication de la demande:
**05.10.83 Bulletin 83/40**

(45) Mention de la délivrance du brevet:
**20.11.86 Bulletin 86/47**

(84) Etats contractants désignés:
**CH DE FR GB LI NL**

(56) Documents cités:
**DE-A-1 913 476**

**Patent Abstracts of Japan vol. 3, no. 46, 18 avril 1979**
**THE JOURNAL OF ORGANIC CHEMISTRY, vol. 45, no. 21, 1980, Columbus, G.C. ACCROMBESSI et al. "Mechanism of the liquid-phase catalytic hydrogenolysis on palladium/carbon of cyclohexene epoxides", pages 4139-4143**

**Chemical Abstracts vol. 70, no. 4, 27 janvier 1969, Columbus, Ohio, USA, H.G. PEER et al. "Synthesis of 4-hydroxy-5-methyl-2,3-dihydro-3-furanone from D-ribose-5-phosphate", page 254, abstract no. 11438v**

(73) Titulaire: **FIRMENICH SA**
**1, route des Jeunes**
**CH-1211 Genève 8 (CH)**

(72) Inventeur: **Whitesides, George M., Prof.**
**124, Grasmere Street**
**Newton, Mass. 02160 (US)**
Inventeur: **Mazenod, François P., Dr.**
**16, Cité Vieusseux**
**CH-1203 Genève (CH)**

(74) Mandataire: **Salvadori, Giuseppe, Dr.**
**c/o Firmenich S.A. Case Postale 239**
**CH-1211 Genève 8 (CH)**

(56) Documents cités:

**Chemical Abstracts vol. 70, no. 4, 27 janvier 1969, Columbus, Ohio, USA, H.G. PEER et al. "Reaction of aldopentoses and secondary amine salts, a convenient method of preparing 4-hydroxy-5-methyl-2,3-dihydro-3-furanone", page 254, abstract no. 11440q**

**THE JOURNAL OF ORGANIC CHEMISTRY, vol. 38, no. 1, 1973, Columbus, G. BÜCHI et al. "Syntheses of 2,5-Dimethyl-4-hydroxy-2,3-dihydrofuran-3-one (Furaneol), a flavor principle of pineapple and strawberry", pages 123-125**

Courier Press, Leamington Spa, England.

EP 0 090 150 B1

## Description

La 4-hydroxy-2,5-diméthyl-2,3-dihydrofuran-3-one, mieux connue dans l'art sous al dénomination de FURANEOL® (marque enregistrée de Firmenich SA, Genève) est un ingrédient aromatisant fort apprécié, notamment lors de la reconstitution de l'arôme typique de la fraise ou de l'ananas. Nombreux ont été les procédés proposés à ce jour pour se préparation. Parmi ceux-ci il convient de mentionner tout particulièrement ceux décrits par L. Re et al. [Helv. Chim. Acta, 56, 1882 (1973)], G. Büchi et E. Demole [J. Org. Chem., 38, 123 (1973)], M. Matsui et al. [Chem. Abstr., 91, 20309 q (1979)], U. Huber et H. J. Wild [demande de brevet européen No. 907] et, plus récemment, C.-H. Ross et al. [demande de brevet européen No. 36'433].

H. G. Peer et G. A. M. van den Ouweland [Recl. Trav. Chim. Pays-Bas, 1968, 87 (1017)] ont décrit un procédé pour la préparation d'un homologue inférieur du FURANEOL®, le 4-hydroxy-5-méthyl-2,3-dihydrofuran-3-one à partir du 5-phosphate D-ribose. Le procédé s'effectue à l'aide d'une base, telle la pyridine, dans un milieu légèrement acide et à une température d'environ 100°C.

La présente invention a pour objet un procédé nouveau et original pour la préparation de FURANEOL®, lequel procédé consiste à soumettre un fructose-1,6-diphosphate ou un fructose-1- ou -6-monophosphate de métal alcalin ou alcalino-terreux à une hydrogénolyse en présence d'un agent basique ou en milieu tamponné.

L'invention est définie dans les revendications.

A titre de fructose-1,6-diphosphate, on peut utiliser par exemple le sel trisodique du D-fructose-1,6-diphosphate (F-1,6-$P_2Na_3$) ou le sel dicalcique du D-fructose-1,6-diphosphate (F-1,6-$P_2Ca_2$). A titre de fructose-1-monophosphate, on peut par contre utiliser le sel de baryum du D-fructose-1-monophosphate (F-1-PBa) ou le sel de sodium du D-fructose-6-phosphate (F-6-$PNa_2$). L'hydrogénolyse a lieu de préférence à une pression supérieure à la pression atmosphérique, les rendements les meilleurs en produit final ayant été en effet observés à une pression comprise entre environ $1,0 \times 10^5$ et $4,0 \times 10^5$ Pa (=15 et 60 psi).

A titre d'agent basique on peut utiliser un hydroxyde de métal alcalin, l'hydroxyde de sodium ou potassium par exemple, ou une base azotée organique, comme par exemple la pipéridine dans un milieu tamponné. Suivant un mode d'exécution préférentiel du procédé de la présente invention, l'hydrogénolyse est effectuée en présence de KOH ou d'acétate de pipéridine, l'hydroxyde de potassium étant employé à raison d'une quantité supérieure à un équivalent par rapport à la quantité de fructose-phosphate de départ.

L'hydrogénolyse est effectuée en présence d'un catalyseur métallique. A cet effet on utilise le platine ou le palladium sur charbon, ce dernier étant employé de préférence.

Quant à la température de réaction, elle peut se situer entre 60 et 120°C, de préférence dans le voisinage de 90°C. A des températures supérieures à la limite indiquée ci-dessus, il devient difficile d'éviter la formation de produits secondaires qui trouvent leur origine dans la décomposition du FURANEOL® déjà formé.

L'hydrogénolyse s'effectue également dans un solvant protique, l'eau par exemple ou l'eau de préférence en mélange avec du méthanol.

Les conditions particulières de réaction seront indiquées ci-après en détail dans les exemples qui suivent (températures en degrés centigrades).

Exemples

L'hydrogénolyse a été effectuée dans un réacteur en verre muni d'un agitateur mécanique. La charge des réactifs s'effectue en y introduisant d'abord le fructose phosphate, puis le solvant, ou mélange de solvants, l'hydroxyde de potassium ou, selon le cas, l'acétate de pipéridine et finalement le catalyseur. Ce dernier peut également être accompagné par un cocatalyseur, tel par exemple l'iodure de sodium.

Avant le procéder à l'introduction d'hydrogène, on effectue le dégazage de la solution résultante au moyen d'argon. Pendant l'hydrogénolyse, on maintient une pression égale à environ 1 à $4 \times 10^5$ Pa. Sous les conditions énoncées, la réaction se complèe après environ 24 heures, la présence de FURANEOL® étant perceptible par la parution d'une coloration jaunâtre du mélange de réaction.

Après filtration, le filtrat clair est extrait avec 4 fractions au moins de chlorure de méthylène, la phase organique étant séparée après adjonction d'une solution aqueuse saturée de chlorure de sodium. Après les traitements usuels de séchage sur du sulfate de magnésium et évaporation sous pression réduite, on obtient le FURANEOL® sous forme d'un solide jaune ayant un point de fusion de 77—9°. Parfois il est obtenu sous forme d'une huile jaunâtre qui cristallise par refroidissement à 4° sous argon. Des échantillᴗns analytiques ont été obtenus par sublimation à 80° et à 9,3 Pa. Les caractères analytiques étaient en tous points identiques à ceux d'un échantillon préparé par l'une des méthodes connues.

Hydrogénolyse du sel calcique de D-fructose-1,6-diphosphate

On a mélangé 10 g de F-1,6-$P_2Ca_2$ (pureté 75%; 19 mmole) avec 1,34 g d'iodure de sodium (8, 9 mmole) dans 50 ml de méthanol et 50 ml d'eau distillée. A ce mélange on a ensuite introduit sous vigoureuse agitation 1,76 g (31,3 mmole) de KOH, puis 200 mg de palladium à 10% sur charbon. Après dégazage à l'argon, on établit à l'intérieur du réacteur une surpression d'environ $4 \times 10^5$ Pa par l'introduction d'$H_2$. Le mélange de réaction a été maintenu ainsi à 94° pendant 24 h, puis le catalyseur métallique et le phosphate inorganique insoluble ont été filtrés et la solution claire jaunâtre extraite. Après les traitements usuels de

2

séchage et évaporation on a obtenu une huile jaune (660 mg, rendement 28,7%) de FURANEOL® brut qui cristallise après avoir été maintenue à 4° sous argon pendant une nuit. Son point de fusion était de 77—79°.

Lorsqu'on a effectué la réaction dans une solution tampon à une pression inférieure, 102 mg (1,2 mmole) de pipéridine, 3 ml de méthanol, 162 mg l'acide acétique glacial (2,7 mmole), 3 ml d'eau, 850 mg (2,09 mmole) de F-1,6-$P_2Na_3$ et 10 mg de palladium à 10% sur charbon ont été mélangés dans le réacteur d'hydrogénolyse. Après dégazage à l'argon, on a établi une pression d'environ $1 \times 10^5$ Pa par l'introduction d'hydrogène, puis le mélange a été maintenu à 96° pendant 27 h. Après les traitements habituels, on a obtenu 110 mg de FURANEOL brut (rendement 41%).

Hydrogénolyse de F-1-PBa

F-1-PBa trihydrate (500 mg; 1,25 mmole) a été soumis à hydrogénolyse comme décrit plus haut en utilisant une solution tampon d'acétate de pipéridine et en présence d'iodure de sodium (32 moles %) dans un mélange 1:1 (v:v) d'eau et méthanol. Après 23 h à 92° sous une pression d'hydrogène égale à environ $1 \times 10^5$ Pa, on a filtré le mélange de réaction et continué comme indiqué plus haut. Après extraction avec du chlorure de méthylène suivie de séchage et évaporation, on a obtenu 20 mg (rendement 14,1%) de FURANEOL® brut.

Hydrogénolyse de F-6-$PNa_2$

635 mg de F-6-$PNa_2$ (2,09 mmoles; pureté 97%) ont été soumis à hydrogénolyse en milieu tamponné dans 10 ml d'eau/méthanol(1:1) suivant le procédé indiqué plus haut. Après 24 h à 96° sous une pression d'hydrogène égale à environ $1 \times 10^5$ Pa. Les traitements habituels ont permis l'isolation de 55 mg de FURANEOL® (rendement 20%).

Les sels phosphoriques de D-fructose, utilisés comme produits de départ dans le procédé décrit ci-dessus, sont des produits commerciaux (source: Sigma Chem. Co.).

Les résultats obtenus au cours de plusieurs essais sont résumés dans le tableau suivant.

TABLEAU

| | Produit de départ | pression d'$H_2$ | solvant | temp. [°C] | temps de réaction [h] | réactif [éq] | NaI [mole %] | rend. [%] |
|---|---|---|---|---|---|---|---|---|
| 1. | F-1,6-$P_2Na_3$ | 60 | MeOH:$H_2O$ | 92 | 24 | 1,6 KOH | 40 | 20 |
| 2. | " | 15 | " | 96 | 27 | Ac-Pipéridine* | — | 35 |
| 3. | F-1,6-$P_2Ca_2$ | 60 | " | 85 | 21 | 1,1 KOH | — | 13 |
| 4. | " | 60 | " | 96 | 24 | 1,7 KOH | — | 33 |
| 5. | " | 60 | " | 95 | 26 | 2 KOH | — | 10 |
| 6. | " | 60 | " | 94 | 24 | 1,7 KOH | 49 | 22 |
| 7. | " | 60 | " | 150 | 6,5 | " | 49 | 25 |
| 8. | " | 60 | $H_2O$ | 96 | 23 | " | 49 | 24 |
| 9. | " | 15 | MeOH:$H_2O$ | 92 | 21 | Ac-Pipéridine* | — | 12 |
| 10. | F-1-PBa | 15 | " | 92 | 23 | " | 32 | 10 |
| 11. | F-6-$PNa_3$ | 15 | " | 96 | 24 | " | — | 20 |
| 12. | " | 40 | " | 80 | 16 | KOH | 23 | 11 |

\* Ac-Pipéridine=Acétate de pipéridine

# 0 090 150

## Revendications

1. Procédé pour la préparation de 4-hydroxy-2,5-diméthyl-2,3-dihydrofuran-3-one, caractérisé en ce qu'on soumet à hydrogénolyse, en milieu basique, un fructose-1,6-diphosphate ou un fructose-1- ou -6-monophosphate de métal alcalin ou alcalino-terreux, ou un de leurs précurseurs, en présence d'un catalyseur métallique constitué par du platine ou du palladium sur charbon.

2. Procédé suivant la revendication 1, caractérisé en ce que l'hydrogénolyse s'effectue en présence de KOH.

3. Procédé suivant la revendication 2, caractérisé en ce que le KOH est utilisé à raison d'environ 1,5 à 2 équivalents par rapport à la quantité pondérale de fructose-1,6-diphosphate ou fructose-1 ou -6-monophosphate soumis à hydrogénolyse.

4. Procédé suivant la revendication 1, caractérisé en ce que l'hydrogénolyse s'effectue en présence d'acétate de pipéridine.

5. Procédé suivant l'une des revendications précédentes, caractérisé en ce que l'hydrogénolyse s'effectue à une pression comprise entre environ 1,0 et $4,0 \times 10^5$ Pa.

6. Procédé suivant l'une des revendications précédentes, caractérisé en ce que l'hydrogénolyse s'effectue à une température comprise entre 60° et 120°C.

7. Procédé suivant l'une des revendications précédentes, caractérisé en ce que l'hydrogénolyse s'effectue dans un solvant protique constitué par de l'eau ou un mélange d'eau et méthanol.

## Patentansprüche

1. Verfahren zur Herstellung von 4-Hydroxy-2,5-dimethyl-2,3-dihydrofuran-3-one, dadurch gekennzeichnet, dass ein Alkali- oder Erdalkali Metall Fructose-1,6-diphosphat oder Fructose-1- oder -6-monophosphat, oder einer ihrer Vorläufer, in Anwesenheit eines Metal Katalysators hydrogenolysiert wird.

2. Verfahren gemäss Anspruch 1, dadurch gekennzeichnet, dass die Hydrogenolyse in Anwesenheit von KOH durchgeführt wird.

3. Verfahren gemäss Anspruch 2, dadurch gekennzeichnet, dass KOH in einer Menge von ungefähr 1,5 bis 2 Aequivalenten, basierend auf dem Gewicht des hydrogenolysierten Fructose-1,6-diphosphat oder Fructose-1- oder -6-monophosphat verwendet wird.

4. Verfahren gemäss Anspruch 1, dadurch gekennzeichnet, dass die Hydrogenolyse in Anwesenheit von Piperidin Acetat durchgeführt wird.

5. Verfahren gemäss einem der vorangehenden Ansprüche, dadurch gekennzeichnet, dass die Hydrogenolyse unter einem Druck von etwa 1,0 bis $4,0 \times 10^5$ Pa durchgeführt wird.

6. Verfahren gemäss einem der vorangehenden Ansprüche, dadurch gekennzeichnet, dass die Hydrogenolyse bei einer Temperatur von 60 bis 120°C durchgeführt wird.

7. Verfahren gemäss einem der vorangehenden Ansprüche, dadurch gekennzeichnet, dass die Hydrogenolyse in einem protischen Lösungsmittel, welches aus Wasser oder einer Mischung von Wasser und Methanol besteht, durchgeführt wird.

## Claims

1. Process for the preparation of 4-hydroxy-2,5-dimethyl-2,3-dihydrofuran-3-one characterized in that an alkali- or alkali-earth metal fructose-1,6-diphosphate or a fructose-1- or -6-monophosphate, or one of its precursors, is subjected to hydrogenolysis in the presence of a metal catalyst.

2. Process according to claim 1, characterized in that the hydrogenolysis is effected in the presence of KOH.

3. Process according to claim 2, characterized in that KOH is employed in an amount of about 1.5 to 2 equivalents based on the weight of the fructose-1,6-diphosphate or fructose-1- or -6-monophosphate subjected to hydrogenolysis.

4. Process according to claim 1, characterized in that the hydrogenolysis is carried out in the presence of piperidine acetate.

5. Process according to one of the preceding claims, characterized in that the hydrogenolysis is carried out at a pressure of between about 1.0 and $4.0 \times 10^5$ Pa.

6. Process according to one of the preceding claims, characterized in that the hydrogenolysis is carried out at a temperature of between 60 and 120°C.

7. Process according to one of the preceding claims, characterized in that the hydrogenolysis is carried out in a protic solvent consisting of water or a mixture of water and methanol.